(19) **Europäisches Patentamt / European Patent Office / Office européen des brevets**

(11) **EP 1 702 557 B1**

(12) **EUROPEAN PATENT SPECIFICATION**

(45) Date of publication and mention
of the grant of the patent:
**19.12.2012 Bulletin 2012/51**

(21) Application number: **06005420.2**

(22) Date of filing: **16.03.2006**

(51) Int Cl.:
*A61B 1/00* (2006.01)          *H04N 1/60* (2006.01)
*H04N 9/67* (2006.01)          *A61B 1/05* (2006.01)
*A61B 1/045* (2006.01)

(54) **Endoscope and image processing device**

Endoskop und Bildverarbeitungseinrichtung

Endoscope et dispositif de traitement d'images

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.03.2005 JP 2005080425**

(43) Date of publication of application:
**20.09.2006 Bulletin 2006/38**

(73) Proprietor: **FUJIFILM Corporation**
**Tokyo (JP)**

(72) Inventors:
• **Takeuchi, Shinji,**
**Fujinon Corporation**
**Saitama-shi**
**Saitama (JP)**

• **Abe, Kazunori,**
**Fujinon Corporation**
**Saitama-shi**
**Saitama (JP)**
• **Ayame, Daisuke,**
**Fujinon Corporation**
**Saitama-shi**
**Saitama (JP)**

(74) Representative: **Klunker . Schmitt-Nilson . Hirsch**
**Patentanwälte**
**Destouchesstrasse 68**
**80796 München (DE)**

(56) References cited:
**EP-A- 1 302 152          US-A- 4 885 634**
**US-A- 5 331 551          US-A- 5 675 378**
**US-A1- 2003 069 471      US-A1- 2004 024 288**

**Description**

FIELD OF THE INVENTION

The present invention relates to an endoscope system apparatus, particularly relates to a constitution used in a medical field for forming and displaying a spectral image (image) containing image information of an arbitrary selected wavelength region.

BACKGROUND OF THE INVENTION

In recent years, in an electronic endoscope apparatus using a solid state image sensor, attention is attracted to spectroscopic imaging combined with a narrow band pass filter, that is, a narrow band filter incorporated electronic endoscope apparatus (Narrow Band Imaging-NBI) based on a prediction of a spectroscopic reflectance in the digesting organ (stomach mucosa or the like). According to the apparatus, three band pass filters of narrow (wavelength) bands are provided in place of a rotating filter of R (red), G (green), B (blue) of a face sequential type, and a spectral image is formed by successively outputting illuminating light by way of the narrow band path filters and processing three signals provided by the illuminating light while changing respective weights thereof similar to a case of R, G, B (RGB) signals. According to the spectral image, in the digesting organ of the stomach, the large intestine or the like, a fine structure which cannot be provided in a background art is extracted.

Meanwhile, it has been proposed to form a spectral image by operation processing based on the image signal provided by white light not by the face sequential type using the narrow band pass filters but by a simultaneous type for arranging a color filter of a small mosaic to a solid state image sensor as shown by JP-A-2003-93336 and Tokyo University Printing Association Foundation 'Analysis and Evaluation of Digital Color Image' by MIYAKE, yolchi(P148 through P153). According thereto, a relationship between respective color sensitivity characteristics of RGB which are formed into numerical value data and a spectroscopic characteristic of a specific narrow band pass filter which is formed into numerical value data is calculated as matrix data (coefficient set) and by operation of the matrix data and RGB signals, a spectral image signal provided by way of the narrow band pass filter is pseudonically provided. When the spectral image is formed by such an operation, it is not necessary to prepare a plurality of filters in correspondence with a desired band pass region, an interchanging arrangement thereof is dispensed with and therefore, large-sized formation of the apparatus is avoided and low cost formation thereof can be achieved.

Meanwhile, according to the endoscope apparatus of the background art, a color image of a taken object is recorded (filed) to an image recording apparatus and also the spectral image can be recorded to the image recording apparatus for data observation. However, in the spectral image, various fine structures can be drawn by selecting a wavelength region thereof and therefore, there is a case in which a plurality or a number of the spectral images to be recorded are provided and in this case, it is necessary to simultaneously record also information constituting a reference. That is, by selecting the wavelength region, various fine structures of, for example, the comparatively bold blood vessel, the capillary, the blood vessel at a deep position, the blood vessel at a shallow position, the cancer tissue having a different progressive degree or the like can be drawn, on the other hand, a difference between specific substances of a difference between oxyhemogrobin and deoxyhemogrobin can also be drawn as a target, further, in order to excellently extract a specific fine structure or the like, it is also necessary to adjust a wavelength region to be selected and the wavelength region becomes important information in forming and observing the spectral images.

Further, the spectral image is formed by constituting an original image by a normal color image, when the spectral image can be compared with the color original image constituting a basis thereof in observing the spectral image, the fine structure of the object is made to be easy to observe and diagnose and an apparatus having an excellent way of use is provided.

Further, when there is a plurality or a number of spectral images to be recorded, sufficient recording operation or the like is desired.

US 4,885,634 discloses an endoscope system apparatus comprising an endoscope including an imaging element that forms a color image of an object; an image recording apparatus in which the color image is recorded and a storing portion that stores data used to form a spectral image. A color processing circuit may perform color processing so as to obtain images in a R mode, a G mode, a B mode and further modes. This prior art system also comprises a matrix circuit which is part of a color adjustment, matrix and calculating circuit used to form e. g. G+B mode and R+B images.

SUMMARY OF THE INVENITON

An object of the present invention is to provide an endoscope system apparatus capable of being made to be easy to observe and diagnose a fine stricture of an object and capable of carrying out an efficient recording operation, by attaching wavelength information important in forming and observing a spectral image in recording the spectral image.

This object is achieved by the features of claim 1.

Preferred embodiments are defined by the dependent claims.

According to that constitution, first, on a side of a processor apparatus including the storing potion and the circuits, in order to calculate $\lambda 1$, $\lambda 2$, $\lambda 3$ signals of wavelength narrow band (component) by matrix operation with regard to RGB signals, matrix data including 61 of wavelength region parameters (coefficient sets p1 through p61) constituted by dividing, for example, a wavelength region from 400nm to 700nm by an interval of 5nm is stored to a memory for operation (a storing portion). Further, when an operator selects three wavelength regions (may be one wavelength region) by wavelength selecting means, matrix data in correspondence with the three wavelength regions is read from the memory, at a spectral image forming circuit, $\lambda 1$, $\lambda 2$, $\lambda 3$ signals are operated from the matrix data and the RGB signals outputted from DSP (digital signal processor) or the like, and the spectral image is formed by the $\lambda 1$, X2, $\lambda 3$ signals. Not only one sheet of the spectral image bur also a plurality of sheets of the spectral images having different wavelength regions can be formed.

Further, in recording operation, the color original image and one sheet or a plurality of sheets of the spectral images are related to each other, both of the color original image and the spectral image (s) are transmitted and recorded from the processor apparatus to the image recording apparatus, and the spectral image is recorded by attaching wavelength information thereto. Further, according to the constitution of claim 3, by one operation of the recording-operation switch, the color original image and one sheet or a plurality of sheets of spectral images (image set) and wave information are recorded to the image recording apparatus.

According to the constitution of claim 4, a plurality of small screens (divided screens) are set on a screen of the display (monitor) provided at the endoscope system apparatus, by the small screens, the color original image and one sheet or a plurality of sheets of the spectral images are simultaneously displayed.

According to an exemplary embodiments of an endoscope system apparatus of the invention, in recording the spectral image, wavelength information thereof is attached and therefore, it is facilitated to observe and diagnose a fine structure of an object, and also information useful for forming the spectral image at a succeeding time is provided. Further, by one operation of the recording-operation switch, for example, by recording the color original image and a plurality of sheets of spectral images simultaneously to the image recording apparatus as a set, sufficient recording operation can be carried out.

## DETAILED DESCRIPTION OF THE DRAWINGS

Fig. 1 is a block diagram showing a constitution of an endoscope system apparatus according to an exemplary embodiment of the invention.

Fig.2 is a view showing a constitution of an operation panel in a processor apparatus of an exemplary embodiment and a wavelength set.

Fig.3 is a graph diagram showing an example of a wavelength region of a spectral image formed in an exemplary embodiment of the invention along with a spectroscopic sensitivity characteristic of a primary color type CCD.

Fig.4 is a graph diagram showing an example of the wavelength region of the spectral image formed in an exemplary embodiment of the invention along with a reflection spectrum.

Fig.5 is a view showing a data content transmitted from the processor apparatus to an image recording apparatus of an exemplary embodiment of the invention.

Figs.6A and 6B illustrate views showing a display state of an original image and a spectral image displayed on a monitor of an exemplary embodiment of the invention.

## DETAILED DESCRIPTION OF THE INVENTION

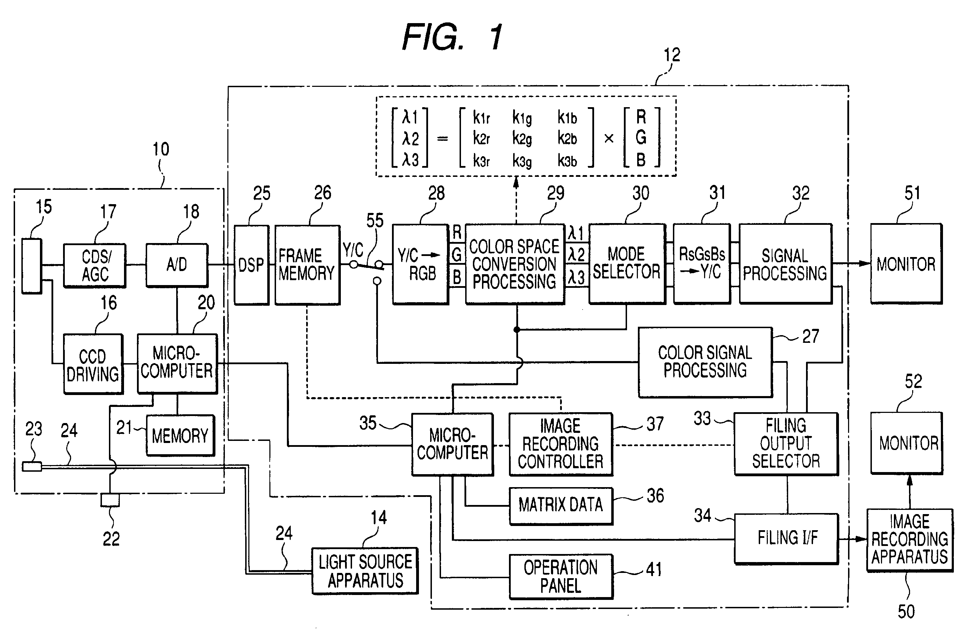

Fig.1 shows a constitution of an electronic endoscope system apparatus according to an exemplary embodiment, and according to the electronic endoscope apparatus, as illustrated, a scope (electronic endoscope) 10 is detachably attached to a processor apparatus 12 and a light source apparatus 14. The processor apparatus 12 is connected with an image recording apparatus 50 and a monitor 51, and the image recording apparatus 50 is connected with a separate monitor 52 used for a reproducing display in observation and diagnosis or the like after inspection. Further, there is also a case in which the light source apparatus 14 is integrally constituted with the processor apparatus 12. The scope 10 is provided with CCD 15 constituting a solid state image sensor (an imaging element) at a front end portion thereof and as the CCD 15, there is used, for example, a complementally color type having color filters of Mg (magenta), Ye (yellow), Cy (cyan), G (green) or a primary color type having color filters of RGB at an imaging face.

The CCD 15 is provided with a CCD driving circuit 16 for forming a drive pulse based on a synchronizing signal and is provided with a CDS/AGC (correlated double sampling/automatic gain control) circuit 17 for sampling and amplifying a picture image (image) signal inputted from the CCD 15, an A/D converter 18. Further, the CCD 15 is arranged with a microcomputer 20 for controlling various circuits in the scope 10 and controlling communication with the processor apparatus 12 (microcomputer 35), and a memory (ROM or the like) 21 for storing information of driving the CCD 15, identifying information of the scope 10 or the like. Further, the scope 10 is provided with a recording operation switch 22 at an operating portion thereof, provided with an illuminating window 23 at a front end portion thereof and the illuminating window 23 is connected to the light source apparatus 14 by a light guide 24.

On the other hand, the processor apparatus 12 is provided with a DSP (digital signal processor) 25 for subjecting an image signal converted into digital to various image processing, the DSP 25 forms and outputs a Y/C signal constituted by a brightness (Y) signal and a chrominance [C(R-Y, B-Y)] signal from an output signal of the CCD 15. Further, the DSP 25 may be arranged on a side of the scope 10. The DSP 25 is connected with the frame memory 26 for storing one frame image outputted from DSP 25 as an original image (Y/C signal) for forming a spectral image. According to the embodiment, a normal image (dynamic picture and stationary picture) and a spectral image (dynamic picture and stationary picture) can selectively be formed and displayed and the frame memory 26 is connected with a color signal processing circuit 27 for forming a normal color image by way of a switch 55 for switching whether a normal image is formed or a spectral image is formed (at one terminal).

Further, other terminal of the switch 55 is provided with a first color converting circuit 28, and at the first color converting circuit 28, the Y (brightness) /C (chrominance) signal outputted from the frame memory 26 is converted into a signal of RGB. A post stage of the first color converting circuit 28 is provided with a color space conversion processing circuit 29 for executing matrix operation for the spectral image and outputting spectral image signals of selected wavelengths $\lambda1$, $\lambda2$, $\lambda3$, a mode selector 30 for selecting either of a spectral image (single color mode) of a single wavelength region (narrow band) and spectral images (three colors mode) comprising three wavelength regions (the mode selector may be provided with two colors mode for selecting two colors), a second color converting circuit 31 for inputting the image signal of the single wavelength region or the image signals of the three wavelength regions ($\lambda1$, $\lambda2$, $\lambda3$) as Rs, Gs, Bs signals for subjecting the image signals to a processing in correspondence with signals of RGB of the background art and converting the Rs, Gs, Bs signals into the Y/C signal, and a signal processing circuit 32 for executing various other signal processing (mirror image processing, mask generation, character generation and the like). Further, the spectral image outputted from the signal processing circuit 32 and the normal color image outputted from the color signal processing circuit 27 are supplied to the monitor 51. The elements 28 to 32 serve as a spectral image-forming circuit.

Further, inside of the processor apparatus 12 of Fig.1 is provided with a filing output selector 33 for inputting a normal color image data outputted from the color image signal processing circuit 27 and spectral image data outputted from the signal processing circuit 32, and a filing I/F (interface) 34 for transmitting image data (stationary picture and dynamic picture) to the image recording apparatus 50. The elements 33 and 34 serve as a data-outputting circuit.

Further, inside of the processor apparatus 12 is provided with the microcomputer 35 for carrying out communication with the scope 10 (microcomputer 20) and controlling respective circuits at inside of the apparatus 12 and reading matrix data from a memory (a storing portion) 36 to provide to the color space conversion processing circuit 29, and an image recording controller 37 for controlling image recording in recording operation. That is, when recording operation is carried out by the recording operation switch 22 of the scope 10, a recording control signal thereof is applied to the image recording controller 37 by way of the microcomputer 20, the microcomputer 35, the image recording controller 37, controls to output data of the normal color image by way of the filing output selector 33 and when the spectral image is selected to form, the image recording controller 37 while controls to output data of the spectral image formed while controlling to store the original image of the frame memory 26 by way of the filing output selector 33 along with the original image.

Further, the memory 36 is stored with matrix (coefficient) data (table) for forming the spectral image based on the RGB signal and according to the embodiment, an example of the matrix data stored to the memory 36 is as shown by Table 1, shown below.

Table 1

| Parameter | $k_{pr}$ | $k_{pg}$ | $k_{pb}$ |
|---|---|---|---|
| p1 | 0.000083 | -0.00188 | 0.003592 |
| : | : | : | : |
| p18 | -0.00115 | 0.000569 | 0.003325 |
| p19 | -0.00118 | 0.001149 | 0.002771 |
| p20 | -0.00118 | 0.001731 | 0.0022 |

(continued)

| Parameter | $k_{pr}$ | $k_{pg}$ | $k_{pb}$ |
|---|---|---|---|
| p21 | -0.00119 | 0.002346 | 0.0016 |
| p22 | -0.00119 | 0.00298 | 0.000983 |
| p23 | -0.00119 | 0.003633 | 0.000352 |
| : | : | : | : |
| p43 | 0.003236 | 0.001377 | -0.00159 |
| p44 | 0.003656 | 0.000671 | -0.00126 |
| p45 | 0.004022 | 0.000068 | -0.00097 |
| p46 | 0.004342 | -0.00046 | -0,00073 |
| p47 | 0.00459 | -0.00088 | -0.00051 |
| p48 | 0.004779 | -0.00121 | -0.00034 |
| p49 | 0.004922 | -0.00148 | -0.00018 |
| p50 | 0.005048 | -0.00172 | -3.6E-05 |
| p51 | 0.005152 | -0.00192 | 0.000088 |
| p52 | 0.005215 | -0.00207 | 0.000217 |
| : | : | : | : |
| p61 | 0.00548 | -0.00229 | 0.00453 |

The matrix data of Table 1 includes 61 of wavelength region parameters (coefficient sets) p1 through p61 constituted by dividing, for example, a wavelength region from 400nm through 700nm by an interval of 5nm, and the parameters pi through p61 are constituted by coefficients $k_{pr}$, $k_{pg}$, $k_{pb}$ (p corresponds to p1 through p61) for matrix operation.

Further, at the color space conversion processing circuit 29, matrix operation of Equation 1, shown below, is carried out by the coefficient $k_{pr}$, $k_{pg}$, $k_{pb}$, and the RGB signal outputted from the first color converting circuit 28.

### Equation 1:

$$\begin{bmatrix} \lambda 1 \\ \lambda 2 \\ \lambda 3 \end{bmatrix} = \begin{bmatrix} k_{1r} & k_{1g} & k_{1b} \\ k_{2r} & k_{2g} & k_{2b} \\ k_{3r} & k_{3g} & k_{3b} \end{bmatrix} \times \begin{bmatrix} R \\ G \\ B \end{bmatrix}$$

That is, when, as $\lambda 1$, $\lambda 2$, $\lambda 3$, for example, parameters p21 (center wavelength 500nm), p45 (center wavelength 620nm), p51 (center wavelength 650nm) of Table 1 are selected, as coefficients ($k_{pr}$, $k_{pg}$, $k_{pb}$), (-0.00119, 0.002346, 0.0016) of p21, (0.004022, 0.000068, -0.00097) of p45, (0.005152, -0.00192, 0.000088) of p51 may be substituted therefor.

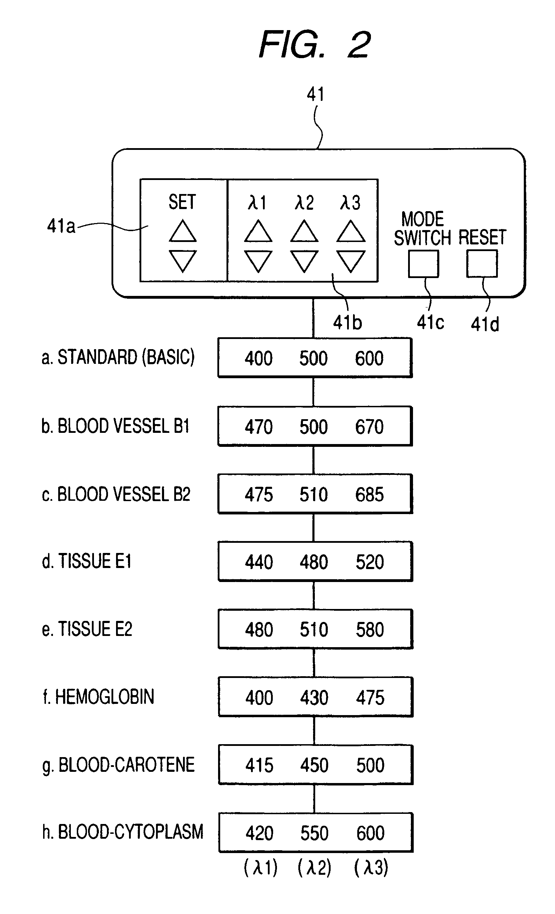

Further, an operation panel 41 of the processor apparatus 12 is arranged with an operation switch for selecting the wavelength region of the spectral image as shown by Fig.2.

In Fig.2, the operation panel 41 is provided with a set selecting (switching) switch (upper and lower switches for successively switching sets in two directions of an alignment) 41a for selecting wavelength sets of a through h or the like (sets of respective center wavelengths), a wavelength selecting switch (upper and lower switches for successively switching selected values in two directions of increase and decrease) for selecting respective wavelength regions (center wavelengths) of the wavelength regions $\lambda 1$, $\lambda 2$, $\lambda 3$, a mode switching switch 41c for switching a single color mode for selecting a single wavelength and a three colors mode, and a reset switch 41d for returning the wavelength region to a standard value and signals of the switches are supplied to the microcomputer 35.

That is, the wavelength selecting switch 41b can select the wavelength regardless of the wavelength region of the wavelength set by the set selecting switch 41a, and can also select to switch the wavelength region by constituting a start position by a value of the wavelength set selected by the set selecting switch 41 a. Further, the microcomputer 35 supplies the matrix data of the wavelength regions λ1, λ2, λ3 selected by signals of the switches 41a through 41d to the color space conversion processing circuit 29. Further, the switching functions can be allocated to keys of a keyboard of the processor apparatus 12 or the like.

The embodiment is constructed by the above-described constitution, first, an explanation will be given of forming the normal image and the spectral image. As shown by Fig.1, in the scope 10, by driving CCD 15 by the CCD driving circuit 16, an image taking signal of an object is outputted from CCD 15, the signal is amplified by correlated double sampling and automatic gain control at CDS/AGC circuit, thereafter, supplied to DSP 25 of the processor apparatus 12 as a digital signal. At the DSP 25, an output signal from the scope 10 is subjected to gamma processing and a color conversion processing to form the Y/C signal comprising the brightness (Y) signal and the chrominance (R-Y,B-Y) signal. An output of the DSP 25 is normally supplied to the color signal processing circuit 27 by the switch 55, subjected to predetermined processing of mirror image processing, mask generation and character generation or the like, thereafter, supplied to the monitor 51 and the normal color image of the object is displayed on the monitor 51

On the other hand, when the recording operation switch 22 of the scope 10 is depressed after selecting the three wavelength regions of λ1, λ2, λ3 signals by operating the operation panel 41 in the spectral image forming mode, a current one frame color image is stored to the frame memory 26 as the original image, the original image (Y/C signal) stored to the frame memory 26 is supplied to the first color converting circuit 28 by the switch 55, and the Y/C signal is converted to the RGB signal by the circuit 28. Next, the RGB signal is supplied to the color space conversion processing circuit 29, and at the color space conversion processing circuit 29, by the RGB signal data and the matrix data, matrix operation of Equation 1, mentioned above, is carried out for forming the spectral image. That is, in forming the spectral image, the microcomputer 35 reads the matrix (coefficient) data in correspondence with the three selected wavelength regions of λ1, λ2, λ3 signals from the memory 36 (Table 1) and the data are supplied to the color space conversion processing circuit 29.

For example, when p21 (center wavelength 500nm), p45 (center wavelength 620nm), p51 (center wavelength 650nm are selected as the three wavelength regions (λ1, λ2, λ3), the signals of λ1, λ2, λ3 are calculated from the RGB signal by matrix operation of Equation 2, shown below.

Equation 2:

$$\begin{bmatrix} \lambda 1 \\ \lambda 2 \\ \lambda 3 \end{bmatrix} = \begin{bmatrix} -0.00119 & 0.002346 & 0.0016 \\ 0.004022 & 0.000068 & -0.00097 \\ 0.005152 & -0.00192 & 0.000088 \end{bmatrix} \times \begin{bmatrix} R \\ G \\ B \end{bmatrix}$$

Further, when the three colors mode is selected by the mode switching switch 41c and the mode selector 30, the signals of λ1, λ2, λ3 are supplied to the second color converting circuit 31 as signals ofRs (=λ1), Gs (=λ2), Bs (=λ3), further, when the single color mode is selected, any of the signals λ1, λ2, λ3 (for example, when the λ2 signal is selected, λ2 signal) is supplied to the second color converting circuit 31 as signals ofRs, Gs, Bs. At the second color converting circuit 31, signals of Rs (=λ1), Gs (=λ2), Bs (=λ3) are converted into the Y/C signal (Y, Rs-Y, Bs-Y) and the Y/C signal is supplied to the monitor 51 by way of the signal processing circuit 32.

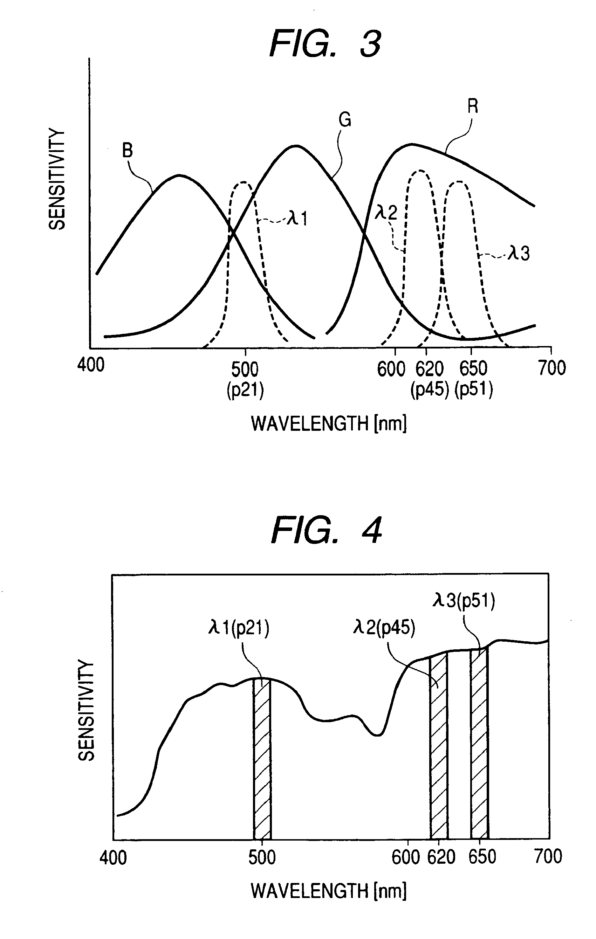

In this way, the spectral image displayed on the monitor 51 is constituted by color components of wavelength regions shown by Fig.3 and Fig.4. That is, Fig.3 is a conceptual diagram constituted by overlapping the three wavelength regions forming the spectral image on a spectroscopic sensitivity characteristic of power filters of CCD 15 (primary color type) (graduations of the color filters and sensitivities of the wavelength regions of λ1, λ2, λ3 signals do not coincide with each other), further, Fig.4 is a conceptual diagram constituted by overlapping the three wavelength regions on a reflection spector of an organism, as illustrated, the wavelengths p21, p45, p51 selected as λ1, λ2, λ3 signals in the embodiment are color signals of wavelength regions constituting center wavelengths successively by 500nm, 620nm, 650nm in a range of about ±10nm, and the spectral image (dynamic picture and stationary picture) constituted by a combination of colors of three wavelength regions is displayed on the monitor 51.

Next, an explanation will be given of selection of the wavelengths of λ1, λ2, λ3 signals. According to embodiment, as shown by Fig.2, as the wavelength sets, for example, there are set and stored a standard (basic) (1) set comprising 400 (center wavelength), 500, 600 [an order of λ1, λ2, λ3 (nm)], blood vessel B1 (b) set of 470, 500, 670 and blood vessel B2 (c) set of 475, 510, 685 for drawing the blood vessels, tissue E1 (d) set of 440, 480, 520 and tissue E2 (e) set of 480, 510, 580 for drawing specific tissues, hemoglobin (f) set of 400, 430, 475 for drawing the difference between oxyhemogrobin and deoxyhemogrobin, blood-carotene (g) set of 415, 450, 500 for drawing a difference between the blood and carotene, 420, 550, 600 (h) set for drawing a difference between the blood and the cytoplasm, and a desired wavelength set can be selected by the set selecting switch 41a therefrom. Thereby, selection of the wavelength set can be facilitated by previously setting frequently used wavelength sets.

Further, when an operator selects an arbitrary wavelength region, when, for example, the standard set a is selected, or the reset switch 41d is depressed, 400, 500, 600 (nm) are displayed on the monitor 51, here, the operator can set the wavelength regions λ1, λ2, λ3 respectively to arbitrary values by operating the wavelength selecting switch 41b. Further, the mode switching switch 41c of Fig.2 is for switching the single color mode and the three colors mode and in the single color mode, all of the wavelength regions λ1, λ2, λ3 are set to the same value such as 470.

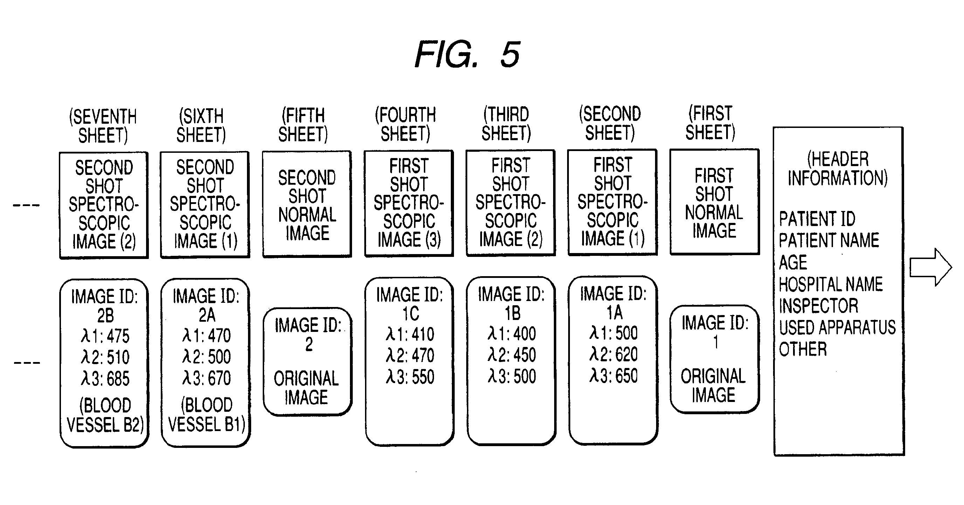

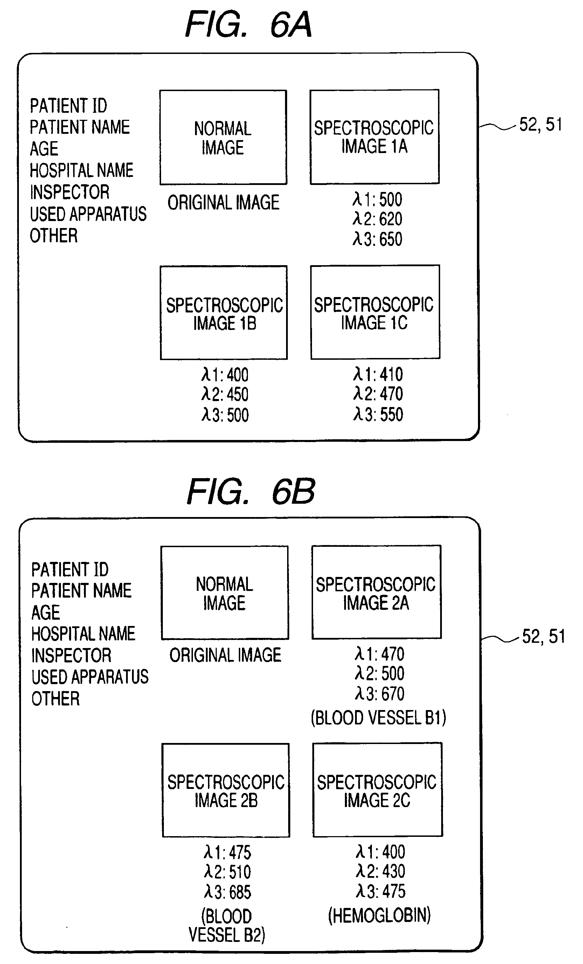

Next, an explanation will be given of a processing of recording the spectral image (stationary picture) to the image recording apparatus 50 in reference to Fig.5 and Figs.6A-6B. Although the spectral image can be recorded by one sheet unit while setting arbitrary wavelength regions, one set may be constituted by a predetermined number of sheets, for example, 3 sheets of spectral images and wavelength regions thereof may previously be set and 4 sheets of images in combination with the original image can be recorded by one time operation of the recording operation switch 22 arranged at the operating portion of the scope 10. Further, in communicating from the processor apparatus 12 to the image recording apparatus 50, the spectral image is related to the original image and the images are added with identification (ID) information of shot number, processing number of the spectral image, set wavelength and the like.

For example, as shown by Fig.5, in communicating the spectral image from the processor apparatus 12, first, information of patient ID, patient name, age, hospital name, inspector, used apparatus or the like (patient information, hospital information, inspection information or the like) is outputted as header information, thereafter, as first shot image data, for example, original image (original) data in which an image ID becomes 1, image ID: 1A and spectral image data (NO.1) added with respective wavelength information of λ1, λ2, λ3 (λ1: 500, λ2: 620, λ3: 650), image ID: 1B and spectral image data (NO.2) added with wavelength information (λ1: 400, λ2: 450, λ3: 500), image ID: 1C and spectral image data (NO.3) added with wavelength information (λ1: 410, λ2: 470, λ3: 550), next, as second shot image data, successive to the original image data in which image ID becomes 2, 2A, 2B, 2C of image ID, spectral image data (for example, packet) added with respective wavelength information of λ1, λ2, λ3, are transmitted to the image recording apparatus 50 by way of the filing output selector 33, the filing I/F 34.

According to the first shot spectral image, the wavelength regions are arbitrarily set, according to the second shot spectral image, as the wavelength regions, blood vessel B 1 (b) set (first sheet), tissue E2 (e) set (second sheet), hemoglobin (f) set (third sheet) of the wavelength sets explained in reference to Fig.2 are selected as the wavelength regions, the image data of the respective shots are communicated by one time operation of the recording operation switch 22 of the scope 10. That is, after selecting the wavelength regions of 3 sheets of the spectral images arbitrarily or by the above-described wavelength sets, 4 sheets of data of the original image and the spectral images are generated by depressing the recording operation switch 22 by one time and is transmitted to the image recording apparatus 50.

Figs.6A and 6B show a display state at the monitor 52 connected to the image recording apparatus 50, in reproducing in observation, diagnosis or the like after inspection, for example, on one screen, 4 sheets of the original image and the spectral images of the first shot is displayed by small screens as shown by Fig.6A, and 4 sheets of the original image and the spectral images of the second shot are displayed by small screens as shown by Fig.6B. Further, the screen is displayed with various information of patient information or the like and displayed with wavelength information (λ1, λ2, λ3) constituting the respective spectral images and the set name or the like in the case of the wavelength set on lower sides of the spectral images or the like. Therefore, in. the spectral images, a target of a fine structure or the like can swiftly and firmly be grasped by wavelength information, the set name.

Further, the monitor 52 of the embodiment can display one sheet of the original image or the spectral image on a total of the screen. That is, in communicating the above-described original image or spectral image, image data formed by the processor apparatus 12 is transmitted as it is without reducing a data amount to a data amount adapted to small screen (divided screen) and a deterioration in an image quality is not brought about even when the image data is displayed on the total of the screen. Further, according to the embodiment, it is possible that the spectral image formed in using the scope 10 is outputted to the monitor 51 by the control of the processor apparatus 12, the monitor 51 can is displayed with a plurality of images on one screen as shown by Figs.6A and 6B, or one sheet of the image is displayed on the total of the screen.

Although according to the above-described explanation, an explanation has been given of the case of recording the stationary picture, the same goes with a case of recording the dynamic picture of the spectral image, and data of wavelength information is communicated to the image recording apparatus 50 along with the dynamic picture of the spectral image.

Although in the above-described embodiment, the wavelength from 400nm to 700nm can be divided into 61 of the wavelength regions to be selected, as the wavelength regions λ1, λ2, λ3, wavelength regions including infrared ray region can also be selected, further, by selecting the wavelength set of λ1, λ2, λ3 in accordance with fluorescence wavelengths, the spectral image constituting a target by a portion of emitting fluorescence may be formed, and by selecting a wavelength region by which a tissue dyed by scattering a colorant can be drawn, a spectral image equivalent to an image in scattering the colorant can also be provided.

**Claims**

1. An endoscope system apparatus comprising:

   an endoscope comprising an imaging element adapted to form a color image of an object;
   an image recording apparatus adapted to record the color image;
   a storing portion storing matrix data of a plurality of wavelength regions for forming a spectral image;
   a spectral image-forming circuit adapted to form a spectral image with respect to a selected wavelength region by a matrix operation using the matrix data of the selected wavelength region in the storing portion and data of a color original image, the matrix operation being

$$\begin{bmatrix} \lambda 1 \\ \lambda 2 \\ \lambda 3 \end{bmatrix} = \begin{bmatrix} k_{1r} & k_{1g} & k_{1b} \\ k_{2r} & k_{2g} & k_{2b} \\ k_{3r} & k_{3g} & k_{3b} \end{bmatrix} \times \begin{bmatrix} R \\ G \\ B \end{bmatrix}$$

   wherein k1r...k3b represent the matrix data, R, G, B represent the color image and $\lambda 1$, $\lambda 2$, $\lambda 3$ represent each a selected wavelength region; and
   a data-outputting circuit adapted to output the spectral image with information of the plurality of wavelength regions which constitutes the spectral image to the image recording apparatus in order to record the spectral image.

2. The endoscope system apparatus according to claim 1, wherein the data-outputting circuit (33, 34) is adapted to output the spectral image along with the color original image to the image recording apparatus (50).

3. The endoscope system apparatus according to claim 1, wherein the data-outputting circuit (33, 34) is adapted to output the color original image and the spectral image to the image recording apparatus (50) by one operation of a recording-operation switch.

4. The endoscope system apparatus according to claim 1, further comprising a display (51, 52) adapted to display the color image of the object, wherein the color original image and the spectral image are simultaneously displayed.

5. An endoscope system apparatus according to claim 1, wherein said data-outputting circuit is adapted to output the wavelength information of the spectral image including identification information of shot number, processing number of the spectral image and set wavelength.

6. The endoscope system apparatus according to claim 1, wherein data of wavelength information is communicated to said image recording apparatus (50) along with the dynamic picture of the spectral image.


**Patentansprüche**

1. Endoskopsystemvorrichtung, umfassend:

   ein Endoskop mit einem Abbildungselement, ausgebildet zum Erzeugen eines Farbbilds eines Objekts;
   eine Bildaufzeichnungsvorrichtung, ausgebildet zum Aufzeichnen des Farbbilds;
   einen Speicherteil, der Matrixdaten einer Mehrzahl von Wellenlängenbereichen zum Erzeugen eines Spektralbilds speichert;
   eine Spektralbilderzeugungsschaltung, ausgebildet zum Erzeugen eines Spektralbilds bezüglich eines ausgewählten Wellenlängenbereichs durch eine Matrixoperation unter Verwendung der Matrixdaten des ausgewählten Wellenlängenbereichs in dem Speicherteil und Daten eines Farb-Originalbilds, wobei die Matrixoperation lautet:

$$\begin{bmatrix} \lambda 1 \\ \lambda 2 \\ \lambda 3 \end{bmatrix} = \begin{bmatrix} k_{1r} & k_{1g} & k_{1b} \\ k_{2r} & k_{2g} & k_{2b} \\ k_{3r} & k_{3g} & k_{3b} \end{bmatrix} \times \begin{bmatrix} R \\ G \\ B \end{bmatrix}$$

wobei k1r ... k3b die Matrixdaten bedeutet, R, G, B das Farbbild bedeutet und $\lambda 1$, $\lambda 2$, $\lambda 3$ jeweils einen ausgewählten Wellenlängenbereich repräsentieren; und
eine Datenausgabeschaltung, ausgebildet zum Ausgeben des Spektralbilds mit Information über die mehreren, das Spektralbild ausmachenden Wellenlängenbereiche an die Bildaufzeichnungsvorrichtung, um das Spektralbild aufzuzeichnen.

2. Endoskopsystemvorrichtung nach Anspruch 1, bei dem die Datenausgabeschaltung (33, 34) dazu ausgebildet ist, das Spektralbild zusammen mit dem Farb-Originalbild an die Bildaufzeichnungsvorrichtung (50) auszugeben.

3. Endoskopsystemvorrichtung nach Anspruch 1, bei der die Datenausgabeschaltung (33, 34) dazu ausgebildet ist, das Farb-Originalbild und das Spektralbild durch eine Betätigung eines Aufzeichnungsschalters an die Bildaufzeichnungsvorrichtung (50) auszugeben.

4. Endoskopsystemvorrichtung nach Anspruch 1, weiterhin umfassend eine Anzeige (51, 52), ausgebildet zum Anzeigen des Farbbilds des Objekts, wobei das Farb-Originalbild und das Spektralbild gleichzeitig angezeigt werden.

5. Endoskopsystemvorrichtung nach Anspruch 1, bei der die Datenausgabeschaltung dazu ausgebildet ist, die Wellenlängeninformation des Spektralbilds einschließlich Kennungsinformation der Aufnahmenummer, einer Verarbeitungsnummer des Spektralbilds und der eingestellten Wellenlänge auszugeben.

6. Endoskopsystemvorrichtung nach Anspruch 1, bei der Daten der Wellenlängeninformation an die Bildaufzeichnungsvorrichtung (50) zusammen mit dem dynamischen Abbild des Spektralbilds übermittelt werden.

**Revendications**

1. Système d'endoscope qui comprend :

un endoscope qui comprend un élément d'imagerie adapté pour former une image en couleur d'un objet ;
un appareil d'enregistrement d'image adapté pour enregistrer ladite image en couleur ;
une partie de stockage qui stocke des données de matrice d'une pluralité de zones de longueurs d'ondes afin de former une image spectrale ;
un circuit de formation d'image spectrale adapté pour former une image spectrale par rapport à une zone de longueur d'onde choisie par une opération de matrice en utilisant les données de matrice de la zone de longueur d'onde choisie dans la partie de stockage et les données d'une image en couleur d'origine, ladite opération de matrice étant

$$\begin{bmatrix} \lambda 1 \\ \lambda 2 \\ \lambda 3 \end{bmatrix} = \begin{bmatrix} k_{1r} & k_{1g} & k_{1b} \\ k_{2r} & k_{2g} & k_{2b} \\ k_{3r} & k_{3g} & k_{3b} \end{bmatrix} \times \begin{bmatrix} R \\ G \\ B \end{bmatrix}$$

où k1r...k3b représentent les données de matrice, R, G, B représentent l'image en couleur, et $\Lambda 1$, $\Lambda 2$, $\Lambda 3$

représentent chacun une zone de longueur d'onde choisie ; et

un circuit de sortie de données adapté pour fournir l'image spectrale, avec les informations de la pluralité de zones de longueurs d'ondes qui constituent l'image spectrale, à l'appareil d'enregistrement d'image, de façon à enregistrer ladite image spectrale.

2. Système d'endoscope selon la revendication 1, dans lequel le circuit de sortie de données (33, 34) est adapté pour fournir l'image spectrale avec l'image en couleur d'origine à l'appareil d'enregistrement d'image (50).

3. Système d'endoscope selon la revendication 1, dans lequel le circuit de sortie de données (33, 34) est adapté pour fournir l'image en couleur d'origine et l'image spectrale à l'appareil d'enregistrement d'image (50) à l'aide d'une seule opération d'un bouton d'enregistrement.

4. Système d'endoscope selon la revendication 1, qui comprend en outre un afficheur (51, 52) adapté pour afficher l'image en couleur de l'objet, dans lequel l'image en couleur d'origine et l'image spectrale sont affichées simultanément.

5. Système d'endoscope selon la revendication 1, dans lequel ledit circuit de sortie de données est adapté pour fournir les informations de longueur d'onde de l'image spectrale, qui comprennent des informations d'identification du nombre de prises de vue, du nombre de traitements de l'image spectrale, et la longueur d'onde définie.

6. Système d'endoscope selon la revendication 1, dans lequel les données des informations de longueur d'onde sont communiquées audit appareil d'enregistrement d'image (50) avec l'image dynamique de l'image spectrale.

FIG. 1

# FIG. 2

41

41a

SET
△
▽

λ1   λ2   λ3
△   △   △
▽   ▽   ▽

MODE
SWITCH   RESET
□   □

41b   41c   41d

| | (λ1) | (λ2) | (λ3) |
|---|---|---|---|
| a. STANDARD (BASIC) | 400 | 500 | 600 |
| b. BLOOD VESSEL B1 | 470 | 500 | 670 |
| c. BLOOD VESSEL B2 | 475 | 510 | 685 |
| d. TISSUE E1 | 440 | 480 | 520 |
| e. TISSUE E2 | 480 | 510 | 580 |
| f. HEMOGLOBIN | 400 | 430 | 475 |
| g. BLOOD-CAROTENE | 415 | 450 | 500 |
| h. BLOOD-CYTOPLASM | 420 | 550 | 600 |

# FIG. 3

# FIG. 4

## FIG. 5

| (SEVENTH SHEET) | (SIXTH SHEET) | (FIFTH SHEET) | (FOURTH SHEET) | (THIRD SHEET) | (SECOND SHEET) | (FIRST SHEET) | |
|---|---|---|---|---|---|---|---|
| SECOND SHOT SPECTRO-SCOPIC IMAGE (2) | SECOND SHOT SPECTRO-SCOPIC IMAGE (1) | SECOND SHOT NORMAL IMAGE | FIRST SHOT SPECTRO-SCOPIC IMAGE (3) | FIRST SHOT SPECTRO-SCOPIC IMAGE (2) | FIRST SHOT SPECTRO-SCOPIC IMAGE (1) | FIRST SHOT NORMAL IMAGE | (HEADER INFORMATION)<br><br>PATIENT ID<br>PATIENT NAME<br>AGE<br>HOSPITAL NAME<br>INSPECTOR<br>USED APPARATUS<br>OTHER |

- - -

| IMAGE ID: 2B<br>$\lambda 1: 475$<br>$\lambda 2: 510$<br>$\lambda 3: 685$<br><br>(BLOOD VESSEL B2) | IMAGE ID: 2A<br>$\lambda 1: 470$<br>$\lambda 2: 500$<br>$\lambda 3: 670$<br><br>(BLOOD VESSEL B1) | IMAGE ID: 2<br><br>ORIGINAL IMAGE | IMAGE ID: 1C<br>$\lambda 1: 410$<br>$\lambda 2: 470$<br>$\lambda 3: 550$ | IMAGE ID: 1B<br>$\lambda 1: 400$<br>$\lambda 2: 450$<br>$\lambda 3: 500$ | IMAGE ID: 1A<br>$\lambda 1: 500$<br>$\lambda 2: 620$<br>$\lambda 3: 650$ | IMAGE ID: 1<br><br>ORIGINAL IMAGE |

- - -

EP 1 702 557 B1

## FIG. 6A

PATIENT ID
PATIENT NAME
AGE
HOSPITAL NAME
INSPECTOR
USED APPARATUS
OTHER

| NORMAL IMAGE | SPECTROSCOPIC IMAGE 1A |
| ORIGINAL IMAGE | $\lambda 1: 500$ $\lambda 2: 620$ $\lambda 3: 650$ |

| SPECTROSCOPIC IMAGE 1B | SPECTROSCOPIC IMAGE 1C |
| $\lambda 1: 400$ $\lambda 2: 450$ $\lambda 3: 500$ | $\lambda 1: 410$ $\lambda 2: 470$ $\lambda 3: 550$ |

~ 52, 51

## FIG. 6B

PATIENT ID
PATIENT NAME
AGE
HOSPITAL NAME
INSPECTOR
USED APPARATUS
OTHER

| NORMAL IMAGE | SPECTROSCOPIC IMAGE 2A |
| ORIGINAL IMAGE | $\lambda 1: 470$ $\lambda 2: 500$ $\lambda 3: 670$ (BLOOD VESSEL B1) |

| SPECTROSCOPIC IMAGE 2B | SPECTROSCOPIC IMAGE 2C |
| $\lambda 1: 475$ $\lambda 2: 510$ $\lambda 3: 685$ (BLOOD VESSEL B2) | $\lambda 1: 400$ $\lambda 2: 430$ $\lambda 3: 475$ (HEMOGLOBIN) |

~ 52, 51

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003093336 A

- US 4885634 A